(19) Europäisches Patentamt
European Patent Office
Office européen des brevets

(11) Publication number: **0 279 096 B1**

(12) **EUROPEAN PATENT SPECIFICATION**

(45) Date of publication of patent specification: **07.07.93**

(51) Int. Cl.5: **C07C 205/31**, C07C 217/76

(21) Application number: **87303649.5**

(22) Date of filing: **24.04.87**

(54) **Benzyl ether compound and process for producing the same.**

(30) Priority: **05.02.87 JP 25240/87**
**05.02.87 JP 25241/87**

(43) Date of publication of application:
**24.08.88 Bulletin 88/34**

(45) Publication of the grant of the patent:
**07.07.93 Bulletin 93/27**

(84) Designated Contracting States:
**DE FR GB**

(56) References cited:
**US-A- 4 348 223**

**CHEMICAL ABSTRACTS, vol. 83, no. 11, 15th September 1975, abstract no. 92302q, Columbus, Ohio, US; R.D. CLONEY et al.: "Molecular orbital study of some substituted benzyl propynyl ethers as insecticide synergists"**

**CHEMICAL ABSTRACTS, vol. 96, no. 3, 18th January 1982, page 417, abstract no. 19761c, Columbus, Ohio, US; J. MACEK et al.: "Studies on local anesthetics. LXXIV. Basic esters of o-(m-)(alkoxymethyl)carbanilic acids"**

(73) Proprietor: **KUREHA KAGAKU KOGYO KABUSHIKI KAISHA**
**9-11 Horidome-cho 1-chome Nihonbashi**
**Chuo-ku**
**Tokyo 103(JP)**

(72) Inventor: **Shida, Takafumi**
**2-11-2 Chuo Nishiki-machi**
**Iwaki-shi Fukushima-ken(JP)**
Inventor: **Kubota, Yoshikazu**
**94-1 Kaminakada Nishiki-machi**
**Iwaki-shi Fukushima-ken(JP)**
Inventor: **Ichinose, Isao**
**1-6 Ochiai Nishiki-machi**
**Iwaki-shi Fukushima-ken(JP)**

(74) Representative: **Woods, Geoffrey Corlett et al**
**J.A. KEMP & CO. 14 South Square Gray's Inn**
**London WC1R 5EU (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.6/3.3.1)

## Description

The present invention relates to benzyl ether compounds useful as intermediates in the preparation of 1,2,4-triazole-3-carboxamide herbicides and to the preparation of these intermediates.

A nitrobenzene compound represented by the formula (IV'):

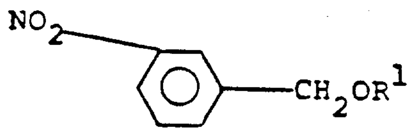

(IV')

wherein $R^1$ represents methyl, ethyl, n-propyl, n-butyl, n-pentyl or n-hexyl, and an aniline compound represented by the formula (V'):

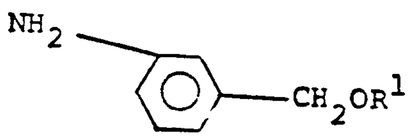

(V')

wherein $R^1$ represents methyl, ethyl, n-propyl, n-butyl, n-pentyl or n-hexyl, are known.

For example, compounds represented by formulae (IV') and (V') wherein $R^1$ is a methyl group are described in The Journal of the Chemical Society, vol. 1954 (1954) page 4127. Compounds represented by formulae (IV') and (V') wherein $R^1$ is methyl are synthesized respectively at a yield of 72% by heating 3-nitrobenzyl chloride in methanol in which metallic sodium is dissolved and at a yield of 82% by reducing 1-(methoxymethyl)-3-nitrobenzene with iron-saline solution. The synthesis of compounds represented by the formula (IV') and (V') wherein $R^1$ is an ethyl group, an n-propyl group, an n-butyl group, an n-pentyl group or an n-hexyl group are described in Chekoslovenska Farmacie, vol. 30 (1981) page 184, in which the compounds represented by the formula (IV') are synthesized at a yield of 65 to 75 % in the same manner as in the synthesis of the compound represented by the formula (IV') wherein $R^1$ is a methyl group, and the aniline compounds represented by the formula (V') are synthesized at a yield of 75 to 81 % by reducing the corresponding nitrobenzene compounds.

As a compound having a branched alkyl group, a compound wherein $R^1$ is an i-propyl group is known.

US-A- 4,348,223 (1982) discloses a compound represented by the formula (IV') wherein $R^1$ is an i-propyl group which is synthesized at a yield of 42 % by heating 3-nitrobenzyl chloride with an aqueous solution of KOH in i-propanol, and further a compound represented by the formula (V') wherein $R^1$ is an i-propyl group which is synthesized at a yield of 91% by catalytic reduction of the corresponding nitrobenzene compound.

Many compounds are known in which $R^1$ is a phenyl group. As a typical example, an unsubstituted phenyl group will be cited. By a process described in The Journal of the Chemical Society, vol. 1954 (1954) page 4573, a compound represented by the formula (IV') wherein $R^1$ is a phenyl group is synthesized at a yield of 60 % by heating 3-nitrobenzyl bromide and phenol in methylethyl ketone in the presence of $K_2CO_3$, and the Journal of Pharmaceutical Sciences vol. 56 (1976) page 871 describes that a compound represented by the formula (V') wherein $R^1$ is a phenyl group is synthesized at a yield of about 50 % by catalytic reduction of the corresponding nitrobenzene derivative.

A compound represented by the formula (IV') wherein $R^1$ is a benzyl group is described in The Journal of Organic Chemistry, vol. 51 (1986) page 2777, and a compound represented by the formula (IV') wherein $R^1$ is a propynyl group ($-CH_2C \equiv CH$) is described in Chemical Abstracts, vol. 83 No. 92302, but the detailed synthetic methods of those compounds are not described.

As a compound represented by the formula (IV') wherein $R^1$ is a fluoroalkyl group a compound represented by the formula (IV') wherein $R^1$ is a 2,2,2-trifluoroethyl group is known. A compound represented by the formula (IV') wherein $R^1$ is a 2,2,2-trifluoroethyl group described in Analytical Chemistry, vol. 54 (1982) page 529 is obtained at a yield of 95 % by reacting 2,2,2-trifluorodiazoethane ($CF_3CH = \overset{+}{N} = \overline{N}$) with 3-nitrobenzyl alcohol

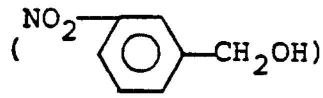

in fluoroboric acid.

However, no prior publication discloses a compound represented by the formula (V) wherein R represents a branched alkyl group of 4 to 8 carbon atoms, a cycloalkyl group of 4 to 8 carbon atoms, an alkyl group of 1 to 3 carbon atoms substituted by an alicyclic group of 3 to 7 carbon atoms, a benzyl group, an alkyl group of 2 to 4 carbon atoms substituted by an alkoxy group of 1 to 4 carbon atoms, an unsaturated alkyl group of 3 to 6 carbon atoms, a straight-chain alkyl group of 2 to 8 carbon atoms which has been substituted by 1 to 15 fluorine atoms or a branched alkyl group of from 3 to 8 carbon atoms substituted by from 1 to 15 fluorine atoms. Further, no prior publication discloses a compound represented by the formula (IV) wherein R' represents a branched alkyl group of 4 to 8 carbon atoms, a cycloalkyl group of 4 to 8 carbon atoms, an alkyl group of 1 to 3 carbon atoms substituted by an alicyclic group of 3 to 7 carbon atoms, an alkyl group of 2 to 4 carbon atoms substituted by an alkoxy group of 1 to 4 carbon atoms, an unsaturated alkyl group of 3 to 6 carbon atoms (propynyl excluded), a straight-chain alkyl group of 3 to 8 carbon atoms which has been substituted by 1 to 15 fluorine atoms or a branched alkyl group of from 3 to 8 carbon atoms substituted by from 1 to 15 fluorine atoms.

As a result of the present inventors' various researches to provide a 1,2,4-triazole-3-carboxamide compound which has an excellent weed killing effect and which is represented by the formula (VI):

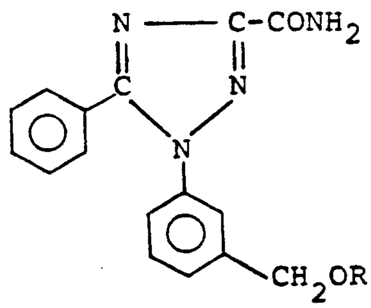

(VI)

wherein R is as defined above, it has been found that a benzyl ether compound represented by the formula (I) is a useful compound as an intermediate compound of the 1,2,4-triazole-3-carboxamide compound represented by the formula (VI) as a herbicide, and on the basis of this finding, the present invention has been attained.

In a first aspect of the present invention, there is provided a benzyl ether compound represented by the general formula (I):

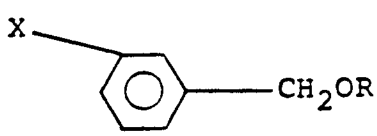

(I)

wherein R represents a branched alkyl group of 4 to 8 carbon atoms, a cycloalkyl group of 4 to 8 carbon atoms, an alkyl group of 1 to 3 carbon atoms substituted by an alicyclic group of 3 to 7 carbon atoms, a benzyl group, an alkyl group of 2 to 4 carbon atoms substituted by an alkoxy group of 1 to 4 carbon atoms, an unsaturated alkyl group of 3 to 6 carbon atoms, a straight-chain alkyl group of 2 to 8 carbon atoms substituted by 1 to 15 fluorine atoms, or a branched alkyl group of 3 to 8 carbon atoms substituted by 1 to 15 fluorine atoms, and X represents a nitro group or an amino group; provided that, when X is a nitro group, R is not a benzyl group, an ethyl group substituted by fluorine atom(s) or a propynyl group.

In a second aspect of the present invention, there is provided a process for preparing a nitrobenzene compound which is represented by the general formula (IV):

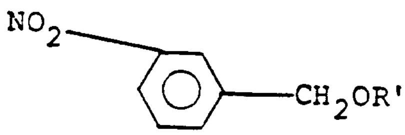

(IV)

wherein R' represents a branched alkyl group of 4 to 8 carbon atoms, a cycloalkyl group of 4 to 8 carbon atoms, an alkyl group of 1 to 3 carbon atoms substituted by an alicyclic group of 3 to 7 carbon atoms, an alkyl group of 2 to 4 carbon atoms substituted by an alkoxy group of 1 to 4 carbon atoms, an unsaturated alkyl group of 3 to 6 carbon atoms, a straight-chain alkyl group of 2 to 8 carbon atoms substituted by 1 to 15 fluorine atoms or a branched alkyl group of 3 to 8 carbon atoms substituted by 1 to 15 fluorine atoms, provided that R' is not an ethyl group substituted by fluorine atom(s) or a propynyl group which comprises reacting a 3-nitrobenzyl chloride represented by the general formula (II):

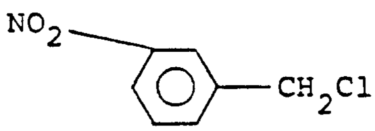

(II)

with an alcohol represented by the general formula (III):

R'OH (III)

wherein R' is as defined above, in an aprotic polar solvent in the presence of an acceptor of hydrogen chloride.

In a third aspect of the present invention, there is provided a process for producing an aniline compound represented by the general formula (V):

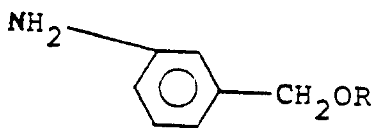

(V)

wherein R is as defined above, which comprises reacting a 3-nitrobenzyl chloride represented by the general formula (II):

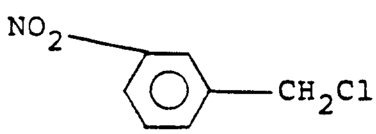

(II)

with an alcohol represented by the following general formula (III'):

ROH (III')

wherein R is as defined above, in an aprotic polar solvent in the presence of an acceptor of hydrogen chloride and reducing the thus obtained nitrobenzene compound.

A preferred compound represented by the general formula (I) is a compound in which R is a branched alkyl group of 4 to 6 carbon atoms, an alkyl group of 1 or 2 carbon atoms substituted by an alicyclic group of 3 to 6 carbon atoms, a benzyl group, an alkyl group of 2 carbon atoms substituted by an alkoxy group of 4 carbon atoms, an unsaturated alkyl group of 3 to 6 carbon atoms, a straight-chain alkyl group of 2 to 8 carbon atoms substituted by 3 to 12 fluorine atoms or a branched alkyl group of 3 to 8 carbon atoms substituted by 3 to 12 fluorine atoms. More specifically, a preferred compound is exemplified by the

following compounds:
1-(2-methylbutoxy)methyl-3-nitrobenzene
1-(3-methylbutoxy)methyl-3-nitrobenzene
1-(2,2-dimethylpropoxy)methyl-3-nitrobenzene
1-(cyclohexylmethoxy)methyl-3-nitrobenzene
1-(2,2,3,3,3-pentafluoropropoxy)methyl-3-nitrobenzene
1-(2,2,3,4,4,4-hexafluorobutoxy)methyl-3-nitrobenzene
1-(2,2,3,3,4,4,4-heptafluorobutoxy)methyl-3-nitrobenzene
1-(2,2,3,3,4,4,5,5-octafluoropentoxy)methyl-3-nitrobenzene
3-[(2-methylbutoxy)methyl]aniline
3-[(3-methylbutoxy)methyl]aniline
3-[(2,2-dimethylpropoxy)methyl]aniline
3-[(cyclohexylmethoxy)methyl]aniline
3-[2,2,2-trifluoroethoxy)methyl]aniline
3-[(2,2,3,3,3-pentafluoropropoxy)methyl]aniline
3-[(2,2,3,4,4,4-hexafluorobutoxy)methyl]aniline
3-[(2,2,3,3,4,4,4-heptafluorobutoxy)methyl]aniline
3-[(2,2,3,3,4,4,5,5-octafluoropentoxy)methyl]aniline.

When an alcohol represented by the formula (III) or (III') is reacted with 3-nitrobenzyl chloride represented by the formula (II) in the processes of the present invention, 1 to 5 equivalents of alcohol per 3-nitrobenzyl chloride is generally used.

As the aprotic polar solvent, a ketone solvent such as acetone and methylethyl ketone; an ether solvent such as tetrahydrofuran, dioxane, ethylene glycol dimethyl ether and diethylene glycol dimethyl ether; a nitrile solvent such as acetonitrile; an amide solvent such as dimethylformamide, dimethylacetamide, N-methylpyrrolidone and hexamethylphosphoramide; and a sulfur containing solvent such as dimethyl sulfoxide and sulfolane may be exemplified.

As the acceptor of hydrogen chloride, an inorganic base such as sodium hydroxide, potassium hydroxide, sodium carbonate, potassium carbonate, sodium hydride and metallic sodium; and an organic base such as triethylamine, pyridine and N,N-dimethylaniline may be exemplified.

The reaction temperature is not lower than the melting point of a solvent or in the range of -10 to 150 °C, preferably not lower than the melting point of a solvent or in the range of 10 to 80 °C.

The reaction is generally completed in 0.1 to 10 ° hours, and thereafter the product is separated by recovering the solvent or pouring water into the reaction mixture.

A conventional reducing method may be adopted to reduce a nitrobenzene compound represented by the formula (IV).

For example, a method of reacting iron, zinc, tin or ferrous salt, stannous salt in an acid, alkaline or neutral solvent may be used. In this case, as the acid solvent, hydrochloric acid, sulfuric acid and acetic acid may be exemplified. As the alkaline solvent alcoholic sodium hydroxide, ethanolic potassium hydroxide, or aqueous ammonia may be exemplified. As the neutral solvent, an aqueous solution with salt, ammonium chloride, or potassium chloride dissolved therein may be exemplified.

As another method, a method of reacting a sulfide such as sodium sulfide,ammonium sulfide, sodium polysulfide and sodium hydrosulfide in an organic solvent such as ethanol and dioxane, an aqueous solvent thereof, or aqueous ammonia may be used. As still another method, a method of reacting colloidal sulfur in an organic solvent such as methanol, ethanol, acetone and dioxane or an aqueous solvent thereof under the coexistence of sodium hydroxide, potassium hydroxide and ammonia may be used.

Still another method, a method of using hydrazine as a hydrogen donor may be adopted. In this case, a high boiling solvent such as diethylene glycol is used as a solvent,reduction is carried out in ethanol in which ferric salt and active carbon coexist, or reduction is carried out in ethanol in which palladium carbon exists.

Furthermore, a catalytic reduction method may also be adopted. In this case, reduction is carried out in ethanol or acetic acid by pressurizing hydrogen to a pressure ranging from a normal pressure to 5 atm in the presence of Raney nickel, palladium carbon, platinum oxide or the like as the catalyst.

The examples of the novel compounds obtained in this manner will be shown together with the physical and chemical properties and the synthetic yields thereof in Tables 1 and 2. Tables 3 and 4 show the results of the elemental analysis of these novel compounds.

According to a process of the present invention, the intended products are obtained at a yield of 80 to 96 % except when there is a steric hindrance by using a secondary alcohol and a primary alcohol having a

ramification at a carbon at the 2-position as an alcohol represented by the formula (III). Thus, this process is superior to a conventional method.

Table 1 Physical and Chemical Properties of

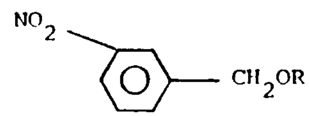

| Compound No. | R | Synthesizing Method * Yield | Boiling point °C (mm Hg) | Nuclear magnetic resonance spectrum ($CDCl_3$, δ, ppm, 60 MHz) |
|---|---|---|---|---|
| 1 | 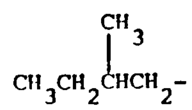 | Method A 65.8 | 119 - 20 (0.18) | 0.90(3H, t, 7.3Hz), 0.94(3H, d, 6.8Hz), 1.17, 1.49, 1.70 (each 1H, m), 3.30 (1H, dd, 6.4, 9.0Hz), 3.38 (1H, dd, 6.4, 9.0Hz), 4.58(2H, s), 7.51(1H, t, 7.8Hz), 7.67(1H, d, 7.8Hz), 8.14(1H, d, 7.8Hz), 8.20(1H, s)** |
| 2 | 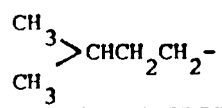 | Method A 90.1 | 116-7 (0.08) | 0.92(6H, d, 6Hz), 1.0-2.0(3H, m), 3.75(2H, t, 6Hz), 4.56(2H, s), 7.3-8.3(4H, m) |
| 3 | 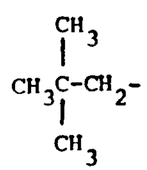 | Method A 68.5 | 97-8 (0.5) | 0.97(9H, s), 3.13(2H, s), 4.56(2H, s), 7.3-8.3(4H, m) |
| 4 | 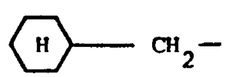 | Method A 65.9 | 128-30 (0.04) | 0.8-1.85(11H, m), 3.07(2H, d, 6.4Hz), 4.57(2H, s), 7.51(1H, t, 7.8Hz), 7.67(1H, d, 7.8Hz), 8.13(1H, d, 7.8Hz), 8.20(1H, s)** |
| 5 | $CH_3(CH_2)_3O(CH_2)_2-$ | Method A 82.3 | 126-7 (0.04) | 0.92(3H, t, 7.3Hz), 1.37(2H, 6-plet, 7.3Hz), 1.59(2H, tt, 7.3, 6.8Hz), 3.48(2H, t, 6.8Hz), 3.66(4H, m), 4.67 (2H, s), 7.51(1H, t, 7.8Hz), 7.69(1H, d, 7.8Hz), 8.14(1H, d, 7.8Hz), 8.23(1H, s)** |

| Compound No. | R | Synthesizing Method * Yield | Boiling point °C (mm Hg) | Nuclear magnetic resonance spectrum ($CDCl_3$, δ, ppm, 60 MHz) |
|---|---|---|---|---|
| 6 | $CH_2$=$CHCH_2$- | Method A 83.2 | 100-1 (0.17) | 4.09(2H, dt, 5.4, 1.5Hz), 4.61(2H, s),5.25(1H, dq, 10.7, 1.5Hz), 5.34 (1H, dq, 17.1, 1.5Hz), 5.97(1H, ddt, 17.1, 10.7, 5.4), 7.52(1H, t, 7.8), 7.69(1H, d, 7.8), 8.15(1H, d, 7.8), 8.22(1H, s)** |
| 7 | $CHF_2CF_2CH_2$- | Method A 96.9 | 83-4 (0.9) | 3.93(2H, t, 13Hz), 4.73(2H, s), 5.97(1H, tt, 54,5), 7.3-8.3(4H, m) |
| 8 | $CF_3CF_2CH_2$- | Method B 83.0 | 86-7 (0.6) | 4.00(2H, t, 13Hz), 4.76(2H, s), 7.3-8.2(4H, m) |
| 9 | $(CF_3)_2$>CH- | Method B 33.5 | 83-4 (0.25) | 4.30(1H, 7-plet, 6Hz), 5.00(2H, s), 7.3-8.2(4H, m) |
| 10 | $CF_3CHFCF_2CH_2$- | Method A 96.8 | 121-3 (0.3) | 3.6-4.2(2H, m), 4.63(2H, s), 5.00(1H, d, 6-plet, 50,7Hz), 7.5-8.4(4H, m) |
| 11 | $CF_3(CF_2)_2CH_2$- | Method A 94.9 | 149-51 (9) | 4.03(2H, tt, 14, 1.5Hz), 4.77(2H, s), 7.3-8.3(4H, m) |
| 12 | $CHF_2(CF_2)_3CH_2$- | Method B 95.0 | 122-4 (0.4) | 3.93(2H, tt, 14, 1.5Hz), 4.67(2H, s), 5.97(1H, tt, 52, 5Hz), 7.2-8.5(4H, m) |

| Compound No. | R | Synthesizing Method * Yield | Boiling point °C (mm Hg) | Nuclear magnetic resonance spectrum ($CDCl_3$, δ, ppm, 60 MHz) |
|---|---|---|---|---|
| 13 | $CHF_2(CF_2)_5CH_2$- | Method A 87.6 | 123-4 (0.6) | 4.20(2H, tt, 14, 1.5Hz), 4.83(2H, s), 5.97(1H, tt, 52, 5Hz), 7.3-8.3(4H, m) |

(Note) * Synthetic Method:

Method A: KOH/dimethylformamide

Method B: NaH/hexamethylphosphoramide

** Measured by a 250 MHz device

Table 2 Physical and Chemical Properties of

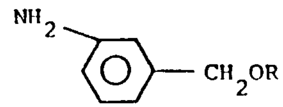

| Compound No. | R | Reducing Method Yield % | Boiling point °C (mm Hg) | Nuclear magnetic resonance spectrum ($CDCl_3$, δ, ppm, 60 MHz) |
|---|---|---|---|---|
| 14 | 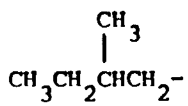 | Method A 97.9 | 102-4 (0.19) | 0.88(3H, t, 7.3Hz), 0.91(3H, d, 6.8Hz), 1.13, 1.48, 1.71 (each 1H, m), 3.22(1H, dd,6.3, 9.0Hz), 3.31(1H, dd, 6.3, 9.0Hz), 3.65(2H, bs), 4.41(2H, s), 6.60(1H, dd, 7.8, 2.0Hz), 6.69(1H, d, 2.0Hz), 6.72(1H, d, 7.8Hz), 7.12(1H, t, 7.8Hz)** |
| 15 | 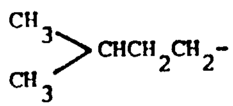 | Method A 97.1 | 105-6 (0.19) | 0.88(6H, d, 6Hz), 1.1-2.2(3H, m), 3.5(2H, bs), 3.47 (2H, t, 6Hz), 4.36(2H, s), 6.5-7.2(4H, m) |
| 16 | 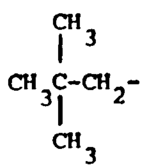 | Method B 96.2 | 98-100 (0.3) | 0.90(9H, s), 3.07(2H, s), 3.53(2H, s), 4.30(2H, s), 6.3-7.3(4H, m) |
| 17 | 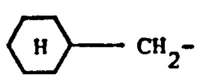 | Method A 91.1 | 137-8 (0.18) | 0.93(2H, m), 1.21(3H, m), 1.68(6H, m), 3.25(2H, d, 6.4Hz), 3.65(2H, bs), 4.41(2H, s), 6.60(1H, dd, 7.8, 2.0Hz), 6.69(1H, d, 2.0Hz), 6.71(1H, d, 7.8Hz), 7.12 (1H, t, 7.8Hz)** |
| 18 | 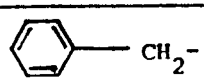 | Method B 92.5 | 140-2 (0.4) | 3.50(2H, bs), 4.40, 4.50(each 2H, s), 6.4-7.4(4H, m), 7.3(5H, s) |

| Compound No. | R | Reducing Method Yield % | Boiling point °C (mm Hg) | Nuclear magnetic resonance spectrum ($CDCl_3$, δ, ppm, 60 MHz) |
|---|---|---|---|---|
| 19 | $CH_3(CH_2)_3O(CH_2)_2$- | Method A 96.9 | 123-5 (0.1) | 0.91(3H, t, 7.3Hz), 1.37(2H, 6-plet, 7.3Hz), 1.58(2H, 5-plet, 7.3Hz), 3.47(2H, t, 7.3Hz), 3.60(4H, s), 3.66(2H, bs), 4.49(2H,s), 6.60(1H, dd, 7.8, 1.5), 6.70(1H, d, 1.5Hz) 6.72(1H, d, 7.8Hz), 7.12(1H, t, 7.8Hz)** |
| 20 | $CH_2 = CHCH_2$- | Method C 72.4 | 88-90 (0.1) | 3.66(2H, bs), 4.02(2H, dt, 5.4, 1.5Hz), 4.44(2H, s), 5.20(1H, dq, 10.3, 1.5Hz), 5.31(1H, dq, 17.1, 1.5Hz), 5.96(1H, ddt, 17.1, 10.3, 5.4Hz), 6.61(1H, dd, 7.8, 1.5Hz), 6.71(1H, d, 1.5Hz), 6.72(1H, d, 7.8Hz), 7.13 (1H, t, 7.8Hz)** |
| 21 | $CF_3CH_2$- | Method B 95.3 | 135-7 (18) | 3.50(2H, bs), 3.70(2H, q, 9Hz), 4.47(2H, s), 6.5-7.2(4H, m) |
| 22 | $CHF_2CF_2CH_2$- | Method B 96.2 | 86-8 (0.3) | 3.50(2H, bs), 3.73(2H, t, 12Hz), 4.47(2H, s), 5.90(1H, tt, 54, 5Hz), 6.4-7.3(4H, m) |
| 23 | $CF_3CF_2CH_2$- | Method D 77.1 | 86-7 (0.6) | 3.60(2H, s), 3.85(2H, t, 13Hz), 4.55(2H, s), 7.47-7.62(4H, m) |
| 24 | 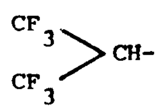 | Method B 90.7 | 113-5 (0.3) | 3.60(2H, bs), 4.07(1H, 7-plet,6Hz), 4.67(2H, s), 6.3-7.3(4H, m) |
| 25 | $CF_3CHFCF_2CH_2$- | Method B 94.5 | 91-3 (0.2) | 3.4-4.1(2H, m), 3.53(2H, bs), 4.48(2H, s), 5.02(1H, d, 6-plet, 50, 7Hz), 6.4-7.3(4H, m) |

| Compound No. | R | Reducing Method Yield % | Boiling point °C (mm Hg) | Nuclear magnetic resonance spectrum ($CDCl_3$, δ, ppm 60 MHz) |
|---|---|---|---|---|
| 26 | $CF_3(CF_2)_2CH_2$- | Method B 97.8 | 82-4 (0.3) | 3.57(2H, s), 3.87(2H, tt, 13, 2Hz), 4.55(2H, s), 6.5-7.4(4H, m) |
| 27 | $CHF_2(CF_2)_3CH_2$- | Method B 92.3 | 104-5 (0.35) | 3.50(2H, s), 3.83(2H, tt, 14, 2Hz), 4.50(2H, s), 6.00(1H, tt, 52, 6Hz), 6.4-7.3(4H, m) |
| 28 | $CHF_2(CF_2)_5CH_2$- | Method B 94.8 | 121-2 (0.9) | 3.47(2H, s), 3.87(2H, t, 14Hz), 4.53(2H, s), 5.97(1H, tt, 52, 5Hz), 6.4-7.3(4H, m) |

(Note) * Reducing method:

Method A: $N_2H_4$/10 % Pd-C/EtOH

Method B: $N_2H_4$/$FeCl_3$-C/EtOH

Method C: Colloidal sulfur/NaOH/Acetone-MeOH

Method D: Fe/Acetic acid

** Measured by a 250 MHz device

Table 3 Elemental Analysis of

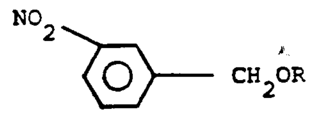

| Compound No. | Molecular formula | Found / Calculated | C(%) | H(%) | N(%) |
|---|---|---|---|---|---|
| 1 | $C_{12}H_{17}NO_3$ | Found | 64.74 | 7.48 | 6.26 |
| | | Calculated | 64.56 | 7.67 | 6.27 |
| 2 | $C_{12}H_{17}NO_3$ | Found | 64.40 | 7.85 | 6.26 |
| | | Calculated | 64.56 | 7.67 | 6.27 |
| 3 | $C_{12}H_{17}NO_3$ | Found | 64.67 | 7.54 | 6.27 |
| | | Calculated | 64.56 | 7.67 | 6.27 |
| 4 | $C_{14}H_{19}NO_3$ | Found | 67.61 | 7.88 | 5.55 |
| | | Calculated | 67.45 | 7.68 | 5.62 |
| 5 | $C_{13}H_{19}NO_4$ | Found | 61.77 | 7.60 | 5.34 |
| | | Calculated | 61.64 | 7.56 | 5.53 |
| 6 | $C_{10}H_{11}NO_3$ | Found | 62.10 | 5.89 | 7.20 |
| | | Calculated | 62.17 | 5.74 | 7.25 |
| 7 | $C_{10}H_9F_4NO_3$ | Found | 44.89 | 3.51 | 5.41 |
| | | Calculated | 44.96 | 3.40 | 5.24 |
| 8 | $C_{10}H_8F_5NO_3$ | Found | 42.16 | 2.83 | 4.73 |
| | | Calculated | 42.12 | 2.83 | 4.91 |
| 9 | $C_{10}H_7F_6NO_3$ | Found | 39.69 | 2.14 | 4.78 |
| | | Calculated | 39.62 | 2.33 | 4.62 |
| 10 | $C_{11}H_9F_6NO_3$ | Found | 41.85 | 2.80 | 4.45 |
| | | Calculated | 41.65 | 2.86 | 4.42 |
| 11 | $C_{11}H_8F_7NO_3$ | Found | 39.22 | 2.23 | 4.02 |
| | | Calculated | 39.42 | 2.41 | 4.18 |

| Compound No. | Molecular formula | Found / Calculated | C(%) | H(%) | N(%) |
|---|---|---|---|---|---|
| 12 | $C_{12}H_9F_8NO_3$ | Found | 39.29 | 2.39 | 3.90 |
| | | Calculated | 39.25 | 2.47 | 3.81 |
| 13 | $C_{14}H_9F_{12}NO_3$ | Found | 36.19 | 2.05 | 2.80 |
| | | Calculated | 35.99 | 1.94 | 3.00 |

Table 4 Elemental Analysis of

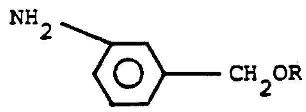

| Compound No. | Molecular formula | Found / Calculated | C(%) | H(%) | N(%) |
|---|---|---|---|---|---|
| 14 | $C_{12}H_{19}NO$ | Found | 74.50 | 9.78 | 7.44 |
| | | Calculated | 74.57 | 9.91 | 7.25 |
| 15 | $C_{12}H_{19}NO$ | Found | 74.76 | 9.96 | 7.15 |
| | | Calculated | 74.57 | 9.91 | 7.25 |
| 16 | $C_{12}H_{19}NO$ | Found | 74.60 | 10.09 | 7.12 |
| | | Calculated | 74.57 | 9.91 | 7.25 |
| 17 | $C_{14}H_{21}NO$ | Found | 76.70 | 9.81 | 6.56 |
| | | Calculated | 76.67 | 9.65 | 6.39 |
| 18 | $C_{14}H_{15}NO$ | Found | 78.87 | 7.28 | 6.63 |
| | | Calculated | 78.84 | 7.09 | 6.57 |
| 19 | $C_{13}H_{21}NO_2$ | Found | 70.12 | 9.58 | 6.25 |
| | | Calculated | 69.92 | 9.48 | 6.27 |
| 20 | $C_{10}H_{13}NO$ | Found | 73.73 | 7.83 | 8.41 |
| | | Calculated | 73.59 | 8.03 | 8.58 |
| 21 | $C_9H_{10}F_3NO$ | Found | 52.53 | 4.73 | 6.68 |
| | | Calculated | 52.69 | 4.91 | 6.83 |
| 22 | $C_{10}H_{11}F_4NO$ | Found | 50.53 | 4.75 | 6.02 |
| | | Calculated | 50.64 | 4.67 | 5.90 |
| 23 | $C_{10}H_{10}F_5NO$ | Found | 46.96 | 4.14 | 5.29 |
| | | Calculated | 47.07 | 3.95 | 5.49 |
| 24 | $C_{10}H_9F_6NO$ | Found | 43.94 | 3.52 | 5.30 |
| | | Calculated | 43.97 | 3.32 | 5.13 |

| Compound No. | Molecular formula | Found / Calculated | C(%) | H(%) | N(%) |
|---|---|---|---|---|---|
| 25 | $C_{11}H_{11}F_6NO$ | Found | 46.20 | 4.02 | 4.68 |
| | | Calculated | 46.00 | 3.86 | 4.88 |
| 26 | $C_{11}H_{10}F_7NO$ | Found | 43.17 | 3.50 | 4.78 |
| | | Calculated | 43.29 | 3.30 | 4.59 |
| 27 | $C_{12}H_{11}F_8NO$ | Found | 42.78 | 3.49 | 4.03 |
| | | Calculated | 42.74 | 3.29 | 4.15 |
| 28 | $C_{14}H_{11}F_{12}NO$ | Found | 38.65 | 2.58 | 3.32 |
| | | Calculated | 38.46 | 2.54 | 3.20 |

A novel benzyl ether compound represented by the general formula (I) which is obtained at a high yield according to the present invention is useful in the synthesis of 1,2,4-triazole-3-carboxamide herbicides represented by the formula (VI) which are described in EP-A-0220956.

Accordingly, the present invention further provides a process for the preparation of a 1,2,4-triazole-3-carboxamide of formula (VI) wherein R is as defined above, which process comprises:

a) reacting an aniline derivative of formula (V) wherein R is as hereinbefore defined with nitrous acid to obtain a diazonium salt;

b) reacting the diazonium salt obtained in step a) with 2-phenyl-2-oxazolin-5-one to obtain a hydrazone compound of formula (VII) wherein R is as hereinbefore defined;

c) reacting the hydrazone compound obtained in step b) with ammonia; and

d) effecting dehydration and ring closure of the compound obtained in step c) to obtain thereby the 1,2,4-triazole-3-carboxamide.

The aniline derivative of formula (V) may be prepared by the reduction of a nitrobenzene compound of formula (IV) wherein R is as defined above.

The advantages as a herbicide of the compound represented by the general formula (VI) are described in detail in EP-A-0220956.

Scheme (I)

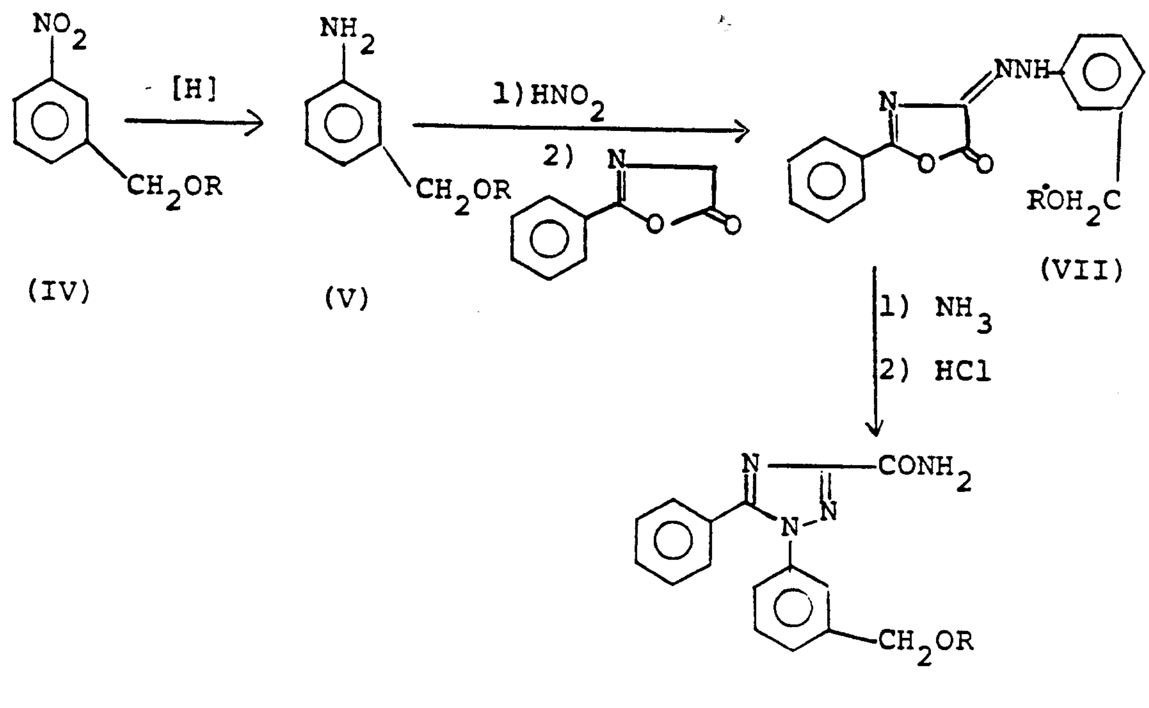


(IV) (V) (VII) (VI)

The present invention will be more precisely explained while referring to Examples as follows.

Example 1: Synthesis of 1-(3-methylbutoxy)methyl-3-nitrobenzene (Compound No. 2)

158.1 g (0.92 mol) of 3-nitrobenzyl chloride was dissolved in a mixture of 500 ml (4.59 mol, 5 equivalents) of 3-methyl-1-butanol and 140 ml of dimethylformamide. To the resultant mixture 78 g (1.39 mol, 1.5 equivalents) of KOH pellets were added while the mixture was being cooled in a water bath and stirred strongly. The reaction temperature was elevated to 43°C and thereafter gradually restored to room temperature. After the mixture was stirred for 7 hours at room temperature, the reaction was completed.

The solid material of the reaction mixture was filtered out and after the pH of the filtrate was adjusted to 2 with dilute hydrochloric acid, the excess alcohol and dimethylformamide were distilled off. The residue was dissolved in a mixed solvent of 450 ml of n-hexane and 50 ml of ethyl acetate, successively washed with 1N-HCl and saturated saline solution, and dried by magnesium sulfate.

After distilling off the solvent, 185.2 g (90.1 %) of the objective product having a boiling point of 116 to 117°C (0.08 mmHg) was obtained by fractional distillation.

Example 2: Synthesis of 1-(2,2,3,3,4,4,4-heptafluorobutoxy)-methyl-3-nitrobenzene (Compound No. 11)

100.0 g (0.58 mol) of 3-nitrobenzyl chloride and 128.3 g (0.62 mol, 1.05 equivalents) of 2,2,3,3,4,4,4-heptafluorobutanol(purity: 96 %) were dissolved in 300 ml of dimethylformamide. To the resultant mixture 49.0 g (0.88 mol, 1.5 equivalents) of KOH pellets were added while the mixture was being cooled in a water bath. The reaction temperature is elevated to 43°C and thereafter gradually restored to room temperature, and the reaction was completed after 30 minutes.

The reaction mixture was diluted with 300 ml of water and after the pH was adjusted to 2 with diluted hydrochloric acid, extraction was carried out two times with a mixed solvent of 250 ml of benzene and 100 ml of hexane. The organic layer was washed with 2N-HCl and saturated saline solution successively, and thereafter was dried by sodium sulfate. After distilling off the solvent, 185.4 g (94.9 %) of the objective product having a boiling point of 149 to 151°C (9 mmHg) was obtained by fractional distillation.

Example 3: Synthesis of 1-(2,2,3,3,3-pentafluoropropoxy)-methyl-3-nitrobenzene (Compound No. 8)

9.6 g (0.24 mol) of sodium hydride (60 % content in mineral oil) was washed with dried n-hexane. Thereafter, it was dispersed into 300 ml of hexamethylphosphoramide. 36.5 g (0.24 mol) of 2,2,3,3,3-pentafluoropropanol was carefully added thereto while being cooled in a water bath to change it into sodium salt. 39.5 g (0.23 mol) of 3-nitrobenzyl chloride dissolved in 50 ml of hexamethylphosphoramide was next added dropwise to the solution.

After 30 minutes from the completion of the dropping, the reaction was completed. The reaction mixture was poured into 200 ml of water. After the pH was adjusted to 2 with diluted hydrochloric acid, extraction was carried out two times with 150 ml of benzene. The organic layer was washed with 100 ml of 6N-HCl three times, washed with water and saturated saline solution successively, and thereafter was dried over anhydrous sodium sulfate.

After distilling off the solvent, 54.5 g (83.0 %) of the objective product having a boiling point of 86 to 87°C (0.6 mmHg) was obtained by fractional distillation.

Example 4: Synthesis of 3-[(3-methylbutoxy)methyl]aniline (Compound No 15)

130 g (0.58 mol) of 1-(3-methylbutoxy)methyl-3-nitrobenzene (Compound No. 2) obtained in Example 1 was dissolved in 150 ml of ethanol, and 0.6 g of 10 % palladium carbon was added thereto. While the mixture was being stirred, 89 ml (1.84 mol) of hydrazine hydrate was added dropwise thereto at a rate which prevents violent foaming. The mixture was refluxed for 3 hours in a water bath after the completion of the dropping, thereby completing the reaction.

After the reaction liquid was left to cool, the catalyst was filtered out and the liquid was washed with ethanol. The filtrate was concentrated and dissolved in 300 ml of dichloromethane. The solution was successively washed with an aqueous solution of 10 % sodium carbonate and saturated saline solution, and was dried by anhydrous potassium carbonate.

After distilling off the solvent, 109.2 g (97.1 %) of the intended product having a boiling point of 105 to 106°C (0.19 mmHg) was obtained by fractional distillation of the residue.

Example 5: Synthesis of 3-[(2,2,3,3,4,4,4-heptafluorobutoxy)methyl]aniline (Compound No. 26)

180 g (0.54 mol) of 1-(2,2,3,3,4,4,4-heptafluorobutoxy)methyl-3-nitrobenzene (Compound No. 11) obtained in Example 2 was dissolved in 500 ml of ethanol, and the mixture was refluxed for 30 minuted after adding 7.9 g of $FeCl_3 \cdot 6H_2O$ and 12.5 g of activated carbon thereto. While the mixture was being stirred, 100 g (2.00 mol) of hydrazine hydrate was added dropwise thereto so as not to cause violent foaming. The mixture was refluxed for 2 hours after the completion of the dropping, thereby completing the reaction. After the reaction mixture was left to cool, the reaction mixture was filtered through a Celite layer and washed with ethanol. The filtrate was concentrated and dissolved in 500 ml of benzene. The solution was successively washed with water and saturated saline solution and was dried by anhydrous sodium sulfate.

After distilling off the solvent, 160.2 g (97.8 %) of the objective product having a boiling point of 82 to 84°C (0.3 mmHg) was obtained by fractional distillation of the residue.

Example 6: Synthesis of 3-[(2,2,3,3,3-pentafluoropropoxy)-methyl]aniline (Compound No. 23)

51.3 g (0.18 mol) of 1-(2,2,3,3,3-pentafluoropropoxy)methyl-3-nitrobenzene (Compound No. 8) obtained in Example 3 was dissolved in 70 ml of acetic acid, and was added dropwise to 150 ml of acetic acid with 70 g of iron powder dispersed therein for 2 hours so as to maintain the reaction temperature at 70 to 80°C. After the completion of the dropping, the reaction mixture was heated to 80°C for 30 minutes in a water bath. After the reaction mixture was left to cool, the reaction mixture was filtered and washed with acetic acid and benzene successively. The filtrate was concentrated and neutralized with an aqueous solution of sodium bicarbonate. Benzene was added thereto and the mixture was filtered again to completely remove the iron salt. The organic layer was washed with saturated saline solution and was dried by anhydrous sodium sulfate.

After distilling off the solvent, 35.4 g (77.1 %) of the objective product having a boiling point of 86 to 87°C (0.6 mmHg) was obtained by fractional distillation. Reference Examples: Synthesis of 1-[3-[(3-methylbutoxy)-methyl]phenyl]-5-phenyl-1H-1,2,4-triazole-3-carboxamide (compound represented by the general formula (VI) wherein R is a 3-methylbutyl group)

(1) Synthesis of 4-[3-[(3-methylbutoxy)methyl]phenylhydrazono]-2-phenyl-2-oxazolin-5-one (compound represented by the general formula (VII) wherein R is a 3-methylbutyl group)

109.2 g (0.566 mol) of 3-[(3-methylbutoxy)methyl]-aniline (Compound No. 15) obtained in Example 4 was dissolved in a mixture of 140 ml of acetic acid and 140 ml of concentrated hydrochloric acid and was cooled in an ice-salt bath. To the solution 39.4 g (0.571 mol) of sodium nitrite dissolved in 100 ml of water was added dropwise at a rate so as to maintain the reaction temperature at not higher than 0 ° C, thereby obtaining a diazonium salt solution.

101.6 g (0.567 mol) of hippuric acid was dispersed in 330 ml (3.49 mol) of acetic anhydride and heated to 80 ° C in a water bath for about 20 minutes, thereby obtaining a solution of 2-phenyl-2-oxazolin-5-one. The thus obtained solution was cooled to -20 ° C in a dry ice acetone bath. Thereto 93 g of anhydrous sodium acetate was added and the diaszonium salt solution prepared in advance was added to the mixture with vigorous stirring at a rate so as to maintain the reaction temperature at not higher than -10 ° C. After the completion of the dropping, the reaction mixture was held at -10 ° C for 2 hours, and thereafter was stirred for 5 hours in an ice bath. Further, the mixture was stirred for one night in a water bath, and thereafter 1.5 l of water was added to filter out a yellow precipitate, which was washed with water to obtain the objective product.

(2) Synthesis of 1-[3-[(3-methylbutoxy)methyl]phenyl]-5-phenyl-1H-1,2,4-triazole-3-carboxamide

The wet crystals of the hydrazone derivative obtained in the reaction in Reference Example (1) was dispersed in 1.3 l of acetone, and 97 ml (0.75 mol) of concentrated aqueous ammonia was added thereto with vigorous stirring. After 1 hour, 78 ml (0.81 mol) of concentrated hydrocloric acid was added to adjust the pH to 2, and the mixture was heated to 50 ° C for 30 minutes in a water bath.

After acetone was distilled off, the residue was extracted two times with 300 ml of benzene, and the organic layer was dried by anhydrous sodium sulfate. The crude crystals obtained by distilling off benzene were recrystallized from n-hexane-dichloromethylene to obtain 128.1 g (yield: 69.1%) of the objective product.

The melting point was from 118 to 120 ° C, which was the same as that of 118 to 120 ° C of a product described in Japanese Patent Application No. 61-239092 (1986).

Test Example

A compound according to the present invention was induced to a final compound (compound represented by the general formula (VI)) having a weed killing activity and described in Japanese Patent Application No. 61-239092 (1986). The final compound was used in the form of a wettable powder. The weed killing activity of each of the final compounds is shown below.

(1) Preparation of a herbicidal composition of a wettable powder

| | |
|---|---|
| Final compound | 50 parts |
| Lignin sulfonate | 5 parts |
| Alkyl sulfonate | 3 parts |
| Diatomaceous earth | 42 parts |

(2) Application test to the soil before germination of plants:

Soil was filled into a planter (650 x 210 x 220 cm) in a state of a crop field. The field was sowed with a predetermined amount of seeds of various test plants, and were thereafter covered with soil. A wettable powder of the above-described herbicidal composition was diluted and was so adjusted that the compound as the effective component was equivalent to 20 g/are. The dilute solution was uniformly dispersed on the surface of the soil, and the plants were grown and controlled in a greenhouse.

Herbicidal effect of the compound on each plant was evaluated on the basis of the following criteria after 25 days from the above-described treatment.

Evaluation Criteria

Killing effect (Herbicidal effect)

0 ..... No weed killing effect (herbicidal effect)

1 ..... not more than 30 % of herbicidal effect

2 ..... 31 to 50 % of herbicidal effect

3 ..... 51 to 70 % of herbicidal effect

4 ..... 71 to 90 % of herbicidal effect

5 ..... 91 to 100 % of herbicidal effect.

(3) The results of the tests are shown in Table 5.

Table 5 Weed Killing Activity (Herbicidal Activity) of Final Compound Represented by the General Formula (VI)

| Test plant | Final compound No.* | | | | | | | | | | | | | | |
|---|---|---|---|---|---|---|---|---|---|---|---|---|---|---|---|
| | 14 | 15 | 16 | 17 | 18 | 19 | 20 | 21 | 22 | 23 | 24 | 25 | 26 | 27 | 28 |
| Echinochloa crus-galli | 5 | 5 | 5 | 5 | 5 | 5 | 5 | 5 | 5 | 5 | 5 | 5 | 5 | 5 | 5 |
| Cyperus iria | 5 | 5 | 5 | 5 | 4 | 5 | 5 | 5 | 5 | 5 | 4 | 5 | 5 | 5 | 5 |
| Bidens pilosa | 5 | 5 | 5 | 5 | 5 | 5 | 5 | 5 | 5 | 5 | 5 | 5 | 5 | 5 | 5 |
| Setaria viridis | 5 | 5 | 5 | 5 | 5 | 5 | 5 | 5 | 5 | 5 | 5 | 5 | 5 | 5 | 5 |
| Solanum nigrum | 5 | 5 | 5 | 5 | 5 | 5 | 5 | 5 | 5 | 5 | 5 | 5 | 5 | 5 | 5 |
| Matricaria chamomilla | 5 | 4 | 4 | 5 | 4 | 4 | 5 | 5 | 5 | 5 | 5 | 5 | 5 | 5 | 5 |
| Amaranthus retyoflexus | 5 | 5 | 5 | 5 | 5 | 5 | 5 | 5 | 5 | 5 | 5 | 5 | 5 | 5 | 5 |
| Triticum aestivum | 0 | 0 | 0 | 0 | 0 | 0 | 0 | 0 | 0 | 0 | 0 | 0 | 0 | 0 | 0 |
| Zea mays | 0 | 0 | 0 | 0 | 0 | 0 | 0 | 0 | 1 | 1 | 0 | 1 | 1 | 1 | 1 |

(Note) * The compound represented by No. corresponds to the compound of the same No. which is represented by the general formula (V) according to the present invention.

## Claims

**1.** A benzyl ether compound represented by the general formula (I)

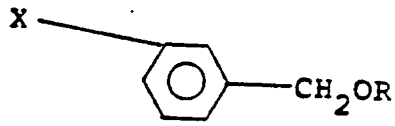

(I)

wherein R represents a branched alkyl group of from 4 to 8 carbon atoms, a cycloalkyl group of from 4 to 8 carbon atoms, an alkyl group of from 1 to 3 carbon atoms substituted by an alicyclic group of from 3 to 7 carbon atoms, a benzyl group, an alkyl group of from 2 to 4 carbon atoms substituted by an alkoxy group of from 1 to 4 carbon atoms, an unsaturated alkyl group of from 3 to 6 carbon atoms, a straight-chain alkyl group of from 2 to 8 carbon atoms substituted by from 1 to 15 fluorine atoms or a branched alkyl group of from 3 to 8 carbon atoms substituted by from 1 to 15 fluorine atoms and X represents a nitro group or an amino group; provided that, when X is a nitro group, R is not a benzyl group, an ethyl group substituted by fluorine atom(s) or a propynyl group.

**2.** A compound according to claim 1, wherein R is a branched alkyl group of from 4 to 6 carbon atoms, an alkyl group of 1 or 2 carbon atoms substituted by an alicyclic group of from 3 to 6 carbon atoms, a benzyl group, an alkyl group of 2 carbon atoms substituted by an alkoxy group of 4 carbon atoms, an unsaturated alkyl group of from 3 to 6 carbon atoms, a straight-chain alkyl group of from 2 to 8 carbon atoms substituted by from 3 to 12 fluorine atoms or a branched alkyl group of from 3 to 8 carbon atoms substituted by from 3 to 12 fluorine atoms.

**3.** A compound according to claim 2, which is 1-(2-methylbutoxy)methyl-3-nitrobenzene.

**4.** A compound according to claim 2, which is 1-(3-methylbutoxy)methyl-3-nitrobenzene.

**5.** A compound according to claim 2, which is 1-(2,2-dimethylpropoxy)methyl-3-nitrobenzene.

**6.** A compound according to claim 2, which is 1-(cyclohexylmethoxy)methyl-3-nitrobenzene.

**7.** A compound according to claim 2, which is 1-(2,2,3,3,3-pentafluoropropoxy)methyl-3-nitrobenzene.

**8.** A compound according to claim 2, which is 1-(2,2,3,4,4,4-hexafluorobutoxy)methyl-3-nitrobenzene.

**9.** A compound according to claim 2, which is 1-(2,2,3,3,4,4,4-heptafluorobutoxy)methyl-3-nitrobenzene.

**10.** A compound according to claim 2, which is 1-(2,2,3,3,4,4,5,5-octafluoropentoxy)methyl-3-nitrobenzene.

**11.** A compound according to claim 2, which is 3-[(2-methylbutoxy)methyl]aniline.

**12.** A compound according to claim 2, which is 3-[(3-methylbutoxy)methyl]aniline.

**13.** A compound according to claim 2, which is 3-[(2,2-dimethylpropoxy)methyl]aniline.

**14.** A compound according to claim 2, which is 3-[(cyclohexylmethoxy)methyl]aniline.

**15.** A compound according to claim 2, which is 3-[(2,2,2-trifluoroethoxy)methyl]aniline.

**16.** A compound according to claim 2, which is 3-[(2,2,3,3,3-pentafluoropropoxy)methyl]-aniline.

**17.** A compound according to claim 2, which is 3-[(2,2,3,4,4,4-hexafluorobutoxy)methyl]-aniline.

**18.** A compound according to claim 2, which is 3-[(2,2,3,3,4,4,4-heptafluorobutoxy)methyl]-aniline.

**19.** A compound according to claim 2, which is 3-[(2,2,3,3,4,4,5,5-octafluoropentoxy)methyl]-aniline.

**20.** A process for preparing a nitrobenzene compound which is represented by the general formula (IV):

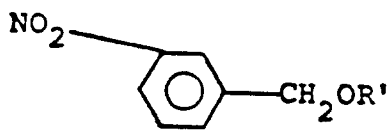

(IV)

wherein R' represents a branched alkyl group of from 4 to 8 carbon atoms, a cycloalkyl group of from 4 to 8 carbon atoms, an alkyl group of from 1 to 3 carbon atoms substituted by an alicyclic group of from 3 to 7 carbon atoms, an alkyl group of from 2 to 4 carbon atoms substituted by an alkoxy group of from 1 to 4 carbon atoms, an unsaturated alkyl group of from 3 to 6 carbon atoms, a straight-chain alkyl group of from 2 to 8 carbon atoms substituted by from 1 to 15 fluorine atoms or a branched alkyl group of from 3 to 8 carbon atoms which has been substituted by from 1 to 15 fluorine atoms, provided that R' is not an ethyl group substituted by fluoroine atoms(s) or a propynyl group; which comprises reacting 3-ntrobenzyl chloride represented by the formula (II):

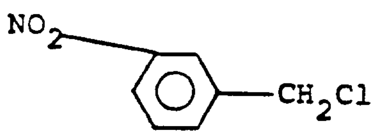

(II)

with an alcohol represented by the general formula (III):

R'OH (III)

wherein R' is as defined above, in an aprotic polar solvent in the presence of an acceptor of hydrogen chloride.

**21.** A process for producing an aniline compound represented by the general formula (V):

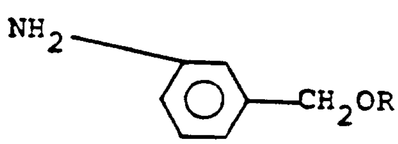

(V)

wherein R is as defined in claim 1; which process comprises reacting 3-nitrobenzyl chloride represented by the formula (II):

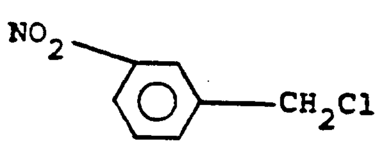

(II)

with an alcohol represented by the following general formula (III'):

ROH (III')

wherein R is as defined above, in an aprotic polar solvent in the presence of an acceptor of hydrogen chloride and reducing the thus obtained nitrobenzene compound.

**22.** A process according to claim 21, wherein the said nitrobenzene compound is reduced by

(1) reaction with iron, zinc, tin, a ferrous salt or a stannous salt in an acid, alkaline or neutral solvent;
(2) reaction with a sulfide in an organic solvent, an aqueous solvent thereof, or aqueous ammonia;
(3) reaction with colloidal sulfur in an organic solvent or an aqueous solvent thereof in the presence of sodium hydroxide, potassium hydroxide or ammonia;
(4) reaction with hydrazine in ethanol in the presence of a ferric salt and active carbon or in ethanol in the presence of palladium carbon; or
(5) catalytic reduction with hydrogen in ethanol or acetic acid in the presence of Raney nickel, palladium carbon or platinum oxide.

**23.** A process according to any one of claims 20 to 22, wherein reaction with the said alcohol is carried out at a temperature of -10°C to 150°C for from 0.1 to 10 hours.

**24.** A process according to any one of claims 20 to 23, wherein the said acceptor of hydrogen chloride is an inorganic base or an organic base.

**25.** A process according to claim 24, wherein the said acceptor of hydrogen chloride is sodium hydroxide, potassium hydroxide, sodium carbonate, potassium carbonate, sodium hydride, metallic sodium, triethylamine, pyridine or N,N-dimethylaniline.

**26.** A process according to any one of claims 20 to 25, wherein the said aprotic polar solvent is a ketone solvent, an ether solvent, a nitrile solvent, an amide solvent or a sulfur-containing solvent.

**27.** A process according to claim 26, wherein the said aprotic polar solvent is acetone, methylethyl ketone, tetrahydrofuran, dioxane, ethylene glycol dimethyl ether, diethylene glycol dimethyl ether, acetonitrile, dimethylformamide, dimethylacetamide, N-methylpyrrolidone, hexamethylphosphoramide, dimethyl sulfoxide or sulfolane.

**28.** A process for the preparation of a 1,2,4-triazole-3-carboxamide of formula (VI)

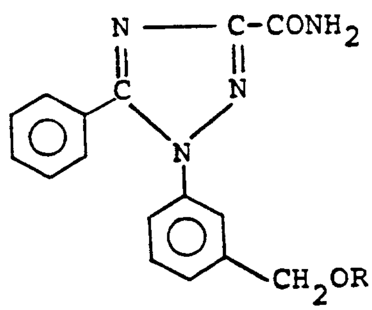

(VI)

wherein R is as defined in claim 1, which process comprises:
a) reacting an aniline derivative of formula (V)

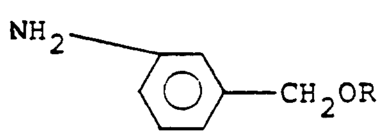

(V)

wherein R is as hereinbefore defined with nitrous acid to obtain a diazonium salt;
b) reacting the diazonium salt obtained in step a) with 2-phenyl-2-oxazolin-5-one to obtain a hydrazone compound of formula (VII)

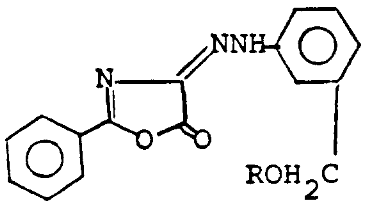

(VII)

wherein R is as hereinbefore defined;

c) reacting the hydrazone compound obtained in step b) with ammonia; and

d) effecting dehydration and ring closure of the compound obtained in step c) to obtain thereby the 1,2,4-triazole-3-carboxamide.

**29.** A process according to claim 28 wherein the aniline derivative of formula (V) is prepared by the reduction of a nitrobenzene compound of formula (IV)

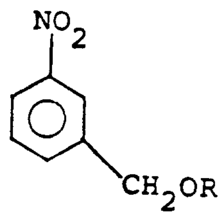

(IV)

wherein R is as defined in claim 1.

**Patentansprüche**

**1.** Benzyletherverbindung der allgemeinen Formel (I)

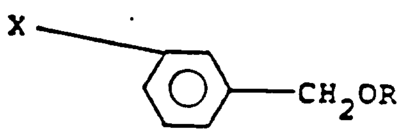

(I)

in der R eine verzweigte Alkylgruppe mit 4 bis 8 Kohlenstoffatomen, eine Cycloalkylgruppe mit 4 bis 8 Kohlenstoffatomen, eine durch eine alicyclische Gruppe mit 3 bis 7 Kohlenstoffatomen substituierte Alkylgruppe mit 1 bis 3 Kohlenstoffatomen, eine Benzylgruppe, eine durch eine Alkoxygruppe mit 1 bis 4 Kohlenstoffatomen substituierte Alkylgruppe mit 2 bis 4 Kohlenstoffatomen, eine ungesättigte Alkylgruppe mit 3 bis 6 Kohlenstoffatomen, eine durch 1 bis 15 Fluoratome substituierte, geradkettige Alkylgruppe mit 2 bis 8 Kohlenstoffatomen oder eine durch 1 bis 15 Fluoratome substituierte, verzweigte Alkylgruppe mit 3 bis 8 Kohlenstoffatomen bedeutet und X eine Nitrogruppe oder eine Aminogruppe bedeutet; mit der Maßgabe, daß, wenn X eine Nitrogruppe bedeutet, R keine Benzylgruppe, keine durch 1 oder mehrere Fluoratome substituierte Ethylgruppe oder keine Propinylgruppe bedeutet.

**2.** Verbindung nach Anspruch 1, wobei R eine verzweigte Alkylgruppe mit 4 bis 6 Kohlenstoffatomen, eine durch eine alicyclische Gruppe mit 3 bis 6 Kohlenstoffatomen substituierte Alkylgruppe mit 1 oder 2 Kohlenstoffatomen, eine Benzylgruppe, eine durch eine Alkoxygruppe mit 4 Kohlenstoffatomen substituierte Alkylgruppe mit 2 Kohlenstoffatomen, eine ungesättigte Alkylgruppe mit 3 bis 6 Kohlenstoffatomen, eine durch 3 bis 12 Fluoratome substituierte, geradkettige Alkylgruppe mit 2 bis 8 Kohlenstoffatomen oder eine durch 3 bis 12 Fluoratome substituierte, verzweigte Alkylgruppe mit 3 bis 8 Kohlenstoffatomen bedeutet.

**3.** Verbindung nach Anspruch 2, nämlich 1-(2-Methylbutoxy)-methyl-3-nitrobenzol.

4. Verbindung nach Anspruch 2, nämlich 1-(3-Methylbutoxy)-methyl-3-nitrobenzol.

5. Verbindung nach Anspruch 2, nämlich 1-(2,2-Dimethylpropoxy)-methyl-3-nitrobenzol.

6. Verbindung nach Anspruch 2, nämlich 1-(Cyclohexylmethoxy)-methyl-3-nitrobenzol.

7. Verbindung nach Anspruch 2, nämlich 1-(2,2,3,3,3-Pentafluorpropoxy)-methyl-3-nitrobenzol.

8. Verbindung nach Anspruch 2, nämlich 1-(2,2,3,4,4,4-Hexafluorbutoxy)-methyl-3-nitrobenzol.

9. Verbindung nach Anspruch 2, nämlich 1-(2,2,3,3,4,4,4-Heptafluorbutoxy)-methyl-3-nitrobenzol.

10. Verbindung nach Anspruch 2, nämlich 1-(2,2,3,3,4,4,5,5-Octafluorpentoxy)-methyl-3-nitrobenzol.

11. Verbindung nach Anspruch 2, nämlich 3-[(2-Methylbutoxy)-methyl]-anilin.

12. Verbindung nach Anspruch 2, nämlich 3-[(3-Methylbutoxy)-methyl]-anilin.

13. Verbindung nach Anspruch 2, nämlich 3-[(2,2-Dimethylpropoxy)-methyl]-anilin.

14. Verbindung nach Anspruch 2, nämlich 3-[(Cyclohexylmethoxy)-methyl]-anilin.

15. Verbindung nach Anspruch 2, nämlich 3-[(2,2,2-Trifluorethoxy)-methyl]-anilin.

16. Verbindung nach Anspruch 2, nämlich 3-[(2,2,3,3,3-Pentafluorpropoxy)-methyl]-anilin.

17. Verbindung nach Anspruch 2, nämlich 3-[(2,2,3,4,4,4-Hexafluorbutoxy)-methyl]-anilin.

18. Verbindung nach Anspruch 2, nämlich 3-[(2,2,3,3,4,4,4-Heptafluorbutoxy)-methyl]-anilin.

19. Verbindung nach Anspruch 2, nämlich 3-[(2,2,3,3,4,4,5,5-Octafluorpentoxy)-methyl]-anilin.

20. Verfahren zur Herstellung einer Nitrobenzol-Verbindung der allgemeinen Formel (IV)

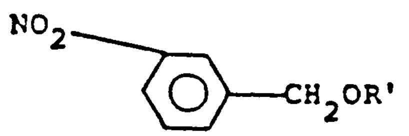

(IV)

in der R' eine verzweigte Alkylgruppe mit 4 bis 8 Kohlenstoffatomen, eine Cycloalkylgruppe mit 4 bis 8 Kohlenstoffatomen, eine durch eine alicyclische Gruppe mit 3 bis 7 Kohlenstoffatomen substituierte Alkylgruppe mit 1 bis 3 Kohlenstoffatomen, eine durch eine Alkoxygruppe mit 1 bis 4 Kohlenstoffatomen substituierte Alkylgruppe mit 2 bis 4 Kohlenstoffatomen, eine ungesättigte Alkylgruppe mit 3 bis 6 Kohlenstoffatomen, eine durch 1 bis 15 Fluoratome substituierte, geradkettige Alkylgruppe mit 2 bis 8 Kohlenstoffatomen oder eine durch 1 bis 15 Fluoratome substituierte, verzweigte Alkylgruppe mit 3 bis 8 Kohlenstoffatomen bedeutet, mit der Maßgabe daß R' keine durch 1 oder mehrere Fluoratome substituierte Ethylgruppe oder keine Propinylgruppe bedeutet; umfassend die Umsetzung von 3-Nitrobenzylchlorid der Formel (II)

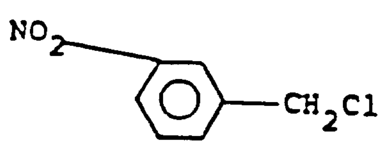

(II)

mit einem Alkohol der allgemeinen Formel (III)

R'OH (III)

in der R' die vorstehend definierte Bedeutung hat, in einem aprotischen polaren Lösungsmittel in Gegenwart eines Akzeptors für Chlorwasserstoff.

**21.** Verfahren zur Herstellung einer Anilinverbindung der allgemeinen Formel (V)

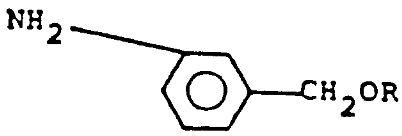

(V)

in der R die in Anspruch 1 definierte Bedeutung hat; umfassend die Umsetzung von 3-Nitrobenzylchlorid der Formel (II)

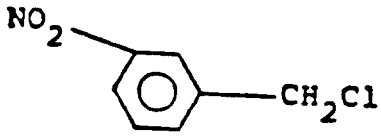

(II)

mit einem Alkohol der allgemeinen Formel (III')

ROH (III')

in der R die vorstehende definierte Bedeutung hat, in einem aprotischen polaren Lösungsmittel in Gegenwart eines Akzeptors für Chlorwasserstoff und die Reduktion der erhaltenen Nitrobenzol-Verbindung.

**22.** Verfahren nach Anspruch 21, wobei die Nitrobenzol-Verbindung reduziert wird durch
(1) Umsetzung mit Eisen, Zink, Zinn, einem Eisen(II)-Salz oder einem Zinn(II)-Salz in einem sauren, alkalischen oder neutralen Lösungsmittel;
(2) Umsetzung mit einem Sulfid in einem organischen Lösungsmittel, einer wäßrigen Lösung davon oder wäßrigem Ammoniak;
(3) Umsetzung mit kolloidalem Schwefel in einem organischen Lösungsmittel oder einer wäßrigen Lösung davon in Gegenwart von Natriumhydroxid, Kaliumhydroxid oder Ammoniak;
(4) Umsetzung mit Hydrazin in Ethanol in Gegenwart eines Eisen(III)-Salzes und Aktivkohle oder in Ethanol in Gegenwart von Palladium-Kohlenstoff; oder
(5) katalytische Reduktion mit Wasserstoff in Ethanol oder Essigsäure in Gegenwart von Raney-Nickel, Palladium-Kohlenstoff oder Platinoxid.

**23.** Verfahren nach einem der Ansprüche 20 bis 22, wobei die Umsetzung mit dem Alkohol 0,1 bis 10 Stunden bei einer Temperatur von -10 bis 150 °C durchgeführt wird.

**24.** Verfahren nach einem der Ansprüche 20 bis 23, wobei es sich bei dem Akzeptor für Chlorwasserstoff um eine anorganische Base oder eine organische Base handelt.

**25.** Verfahren nach Anspruch 24, wobei es sich bei dem Akzeptor für Chlorwasserstoff um Natriumhydroxid, Kaliumhydroxid, Natriumcarbonat, Kaliumcarbonat, Natriumhydrid, metallisches Natrium, Triethylamin, Pyridin oder N,N-Dimethylanilin handelt.

**26.** Verfahren nach einem der Ansprüche 20 bis 25, wobei es sich bei dem aprotischen polaren Lösungsmittel um ein Ketonlösungsmittel, ein Etherlösungsmittel, ein Nitrillösungsmittel, ein Amidlösungsmittel oder ein schwefelhaltiges Lösungsmittel handelt.

**27.** Verfahren nach Anspruch 26, wobei es sich bei dem aprotischen polaren Lösungsmittel um Aceton, Methylethylketon, Tetrahydrofuran, Dioxan, Ethylenglykoldimethylether, Diethylenglykoldimethylether, Acetonitril, Dimethylformamid, Dimethylacetamid, N-Methylpyrrolidon, Hexamethylphosphoramid, Dimethylsulfoxid oder Sulfolan handelt.

**28.** Verfahren zur Herstellung eines 1,2,4-Triazol-3-carboxamids der Formel (VI)

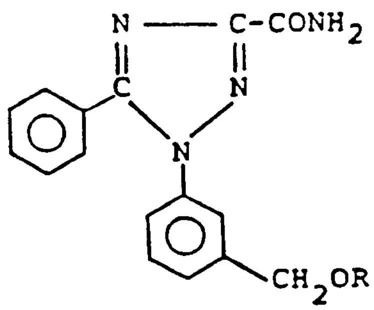

(VI)

in der R die in Anspruch 1 definierte Bedeutung hat, umfassend:

a) die Umsetzung eines Anilinderivats der Formel (V)

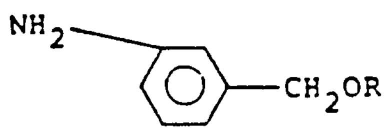

(V)

in der R die vorstehend definierte Bedeutung hat, mit salpetriger Säure unter Bildung eines Diazoniumsalzes;

b) die Umsetzung des in Stufe (a) erhaltenen Diazoniumsalzes mit 2-Phenyl-2-oxazolin-5-on unter Bildung einer Hydrazon-Verbindung der Formel (VII)

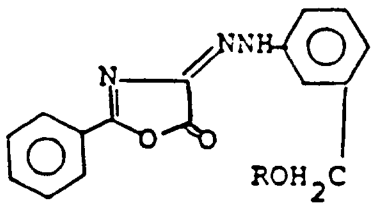

(VII)

in der R die vorstehend definierte Bedeutung hat;

c) die Umsetzung der in Stufe b) erhaltenen Hydrazon-Verbindung mit Ammoniak; und

d) die Durchführung der Dehydratisierung und des Ringschlusses der in Stufe c) erhaltenen Verbindung unter Bildung des 1,2,4-Triazol-3-carboxamids.

**29.** Verfahren nach Anspruch 28, wobei das Anilin-Derivat der Formel (V) durch Reduktion einer Nitrobenzol-Verbindung der Formel (IV)

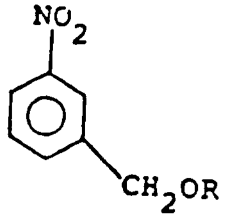

(IV)

in der R die in Anspruch 1 definierte Bedeutung hat, hergestellt wird.

**Revendications**

**1.** Composé éther benzylique, représenté par la formule générale (I)

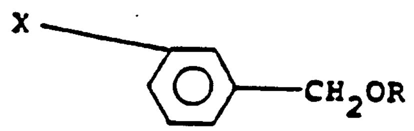

(I)

dans laquelle R représente un groupe alkyle ramifié de 4 à 8 atomes de carbone, un groupe cycloalkyle de 4 à 8 atomes de carbone, un groupe alkyle de 1 à 3 atomes de carbone substitué par un groupe alicyclique de 3 à 7 atomes de carbone, un groupe benzyle, un groupe alkyle de 2 à 4 atomes de carbone substitué par un groupe alcoxy de 1 à 4 atomes de carbone, un groupe alkyle insaturé de 3 à 6 atomes de carbone, un groupe alkyle à chaîne droite de 2 à 8 atomes de carbone substitué par 1 à 15 atomes de fluor, ou un groupe alkyle ramifié de 3 à 8 atomes de carbone substitué par 1 à 15 atomes de fluor, et X représente un groupe nitro ou un groupe amino; à condition que, lorsque X est un groupe nitro, R ne soit pas un groupe benzyle, un groupe éthyle substitué par un ou des atomes de fluor ou un groupe propynyle.

**2.** Composé selon la revendication 1, dans lequel R est un groupe alkyle ramifié de 4 à 6 atomes de carbone, un groupe alkyle de 1 à 2 atomes de carbone substitué par un groupe alicyclique de 3 à 6 atomes de carbone, un groupe benzyle, un groupe alkyle de 2 atomes de carbone substitué par un groupe alcoxy de 4 atomes de carbone, un groupe alkyle insaturé de 3 à 6 atomes de carbone, un groupe alkyle à chaîne droite de 2 à 8 atomes de carbone substitué par 3 à 12 atomes de fluor ou un groupe alkyle ramifié de 3 à 8 atomes de carbone substitué par 3 à 12 atomes de fluor.

**3.** Composé selon la revendication 2, qui est le 1-(2-méthylbutoxy)méthyl-3-nitrobenzène.

**4.** Composé selon la revendication 2, qui est le 1-(3-méthylbutoxy)méthyl-3-nitrobenzène.

**5.** Composé selon la revendication 2, qui est le 1-(2,2-diméthylpropoxy)méthyl-3-nitrobenzène.

**6.** Composé selon la revendication 2, qui est le 1-(cyclohexylméthoxy)méthyl-3-nitrobenzène.

**7.** Composé selon la revendication 2, qui est le 1-(2,2,3,3,3-pentafluoropropoxy)méthyl-3-nitrobenzène.

**8.** Composé selon la revendication 2, qui est le 1-(2,2,3,4,4,4-hexafluorobutoxy)méthyl-3-nitrobenzène.

**9.** Composé selon la revendication 2, qui est le 1-(2,2,3,3,4,4,4-heptafluorobutoxy)méthyl-3-nitrobenzène.

**10.** Composé selon la revendication 2, qui est le 1-(2,2,3,3,4,4,5,5-octafluoropentoxy)méthyl-3-nitrobenzène.

**11.** Composé selon la revendication 2, qui est la 3-[(2-méthylbutoxy)méthyl]aniline.

**12.** Composé selon la revendication 2, qui est la 3-[(3-méthylbutoxy)méthyl]aniline.

**13.** Composé selon la revendication 2, qui est la 3-[(2,2-diméthylpropoxy)méthyl]aniline.

**14.** Composé selon la revendication 2, qui est la 3-[(cyclohexylméthoxy)méthyl]aniline.

**15.** Composé selon la revendication 2, qui est la 3-[(2,2,2-trifluoroéthoxy)méthyl]aniline.

**16.** Composé selon la revendication 2, qui est la 3-[(2,2,3,3,3-pentafluoropropoxy)méthyl]aniline.

**17.** Composé selon la revendication 2, qui est la 3-[(2,2,3,4,4,4-hexafluorobutoxy)méthyl]aniline.

**18.** Composé selon la revendication 2, qui est la 3-[(2,2,3,3,4,4,4-heptafluorobutoxy)méthyl]aniline.

**19.** Composé selon la revendication 2, qui est la 3-[(2,2,3,3,4,4,5,5-octafluoropentoxy)méthyl]aniline.

**20.** Procédé de préparation d'un composé nitrobenzène, représenté par la formule générale (IV) :

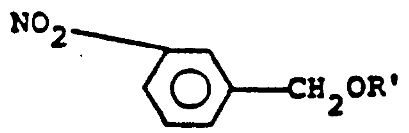

(IV)

dans laquelle R' représente un groupe alkyle ramifié de 4 à 8 atomes de carbone, un groupe cycloalkyle de 4 à 8 atomes de carbone, un groupe alkyle de 1 à 3 atomes de carbone substitué par un groupe alicyclique de 3 à 7 atomes de carbone, un groupe alkyle de 2 à 4 atomes de carbone substitué par un groupe alcoxy de 1 à 4 atomes de carbone, un groupe alkyle insaturé de 3 à 6 atomes de carbone, un groupe alkyle à chaîne droite de 2 à 8 atomes de carbone substitué par 1 à 15 atomes de fluor, ou un groupe alkyle ramifié de 3 à 8 atomes de carbone substitué par 1 à 15 atomes de fluor, à condition que R' ne soit pas un groupe éthyle substitué par un ou des atomes de fluor ou un groupe propynyle, qui comprend la réaction de chlorure de 3-nitrobenzyle représenté par la formule (II) :

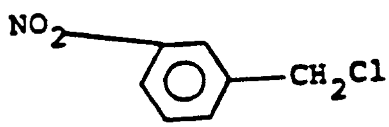

(II)

avec un alcool représenté par la formule générale (III) :

R'OH (III)

dans laquelle R' est tel que défini ci-dessus, dans un solvant polaire aprotique, en présence d'un accepteur de chlorure d'hydrogène.

**21.** Procédé de production d'un composé aniline représenté par la formule générale (V) :

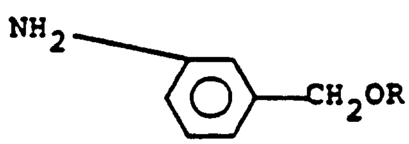

(V)

dans laquelle R est tel que défini dans la revendication 1, ce procédé comprenant la réaction de chlorure de 3-nitrobenzyle représenté par la formule (II) :

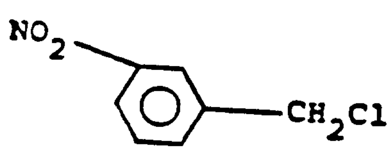

(II)

avec un alcool représenté par la formule générale (III') :

ROH (III')

dans laquelle R est tel que défini ci-dessus, dans un solvant polaire aprotique, en présence d'un accepteur de chlorure d'hydrogène, et la réduction du composé nitrobenzène ainsi obtenu.

**22.** Procédé selon la revendication 21, dans lequel ledit composé nitrobenzène est réduit par
(1) réaction de fer, de zinc, d'étain, d'un sel ferreux ou d'un sel stanneux, dans un solvant acide, alcalin ou neutre;
(2) réaction avec un sulfure dans un solvant organique, un solvant aqueux de celui-ci, ou de l'ammoniaque aqueux;
(3) réaction avec du soufre colloïdal dans un solvant organique, ou un solvant aqueux de celui-ci, en présence d'hydroxyde de sodium, d'hydroxyde de potassium ou d'ammoniac;
(4) réaction avec de l'hydrazine dans de l'éthanol, en présence d'un sel ferrique et de charbon actif, ou dans de l'éthanol en présence de palladium sur charbon; ou
(5) réduction catalytique avec de l'hydrogène, dans de l'éthanol ou del'acide acétique, en présence de nickel de Raney, de palladium sur charbon ou d'oxyde de platine.

**23.** Procédé selon l'une quelconque des revendications 20 à 22, dans lequel la réaction avec ledit alcool est réalisée à une température de -10 °C à 150 °C pendant de 0,1 à 10 heures.

**24.** Procédé selon l'une quelconque des revendications 20 à 23, dans lequel ledit accepteur de chlorure d'hydrogène est une base minérale ou une base organique.

**25.** Procédé selon la revendication 24, dans lequel ledit accepteur de chlorure d'hydrogène est l'hydroxyde de sodium, l'hydroxyde de potassium, le carbonate de sodium, le carbonate de potassium, l'hydrure de sodium, le sodium métallique, la triéthylamine, la pyridine ou la N,N-diméthylaniline.

**26.** Procédé selon l'une quelconque des revendications 20 à 25, dans lequel ledit solvant polaire aprotique est un solvant cétone, un solvant éther, un solvant nitrile, un solvant amide ou un solvant soufré.

**27.** Procédé selon la revendication 26, dans lequel ledit solvant polaire aprotique est l'acétone, la méthyléthylcétone, le tétrahydrofurane, le dioxane, l'éther diméthylique d'éthylèneglycol, l'éther diméthylique de diéthylèneglycol, l'acétonitrile, le diméthylformamide, le diméthylacétamide, la N-méthylpyrrolidone, l'hexaméthylphosphoramide, le diméthylsulfoxyde ou le sulfolane.

**28.** Procédé de préparation d'un 1,2,4-triazole-3-carboxamide de formule (VI)

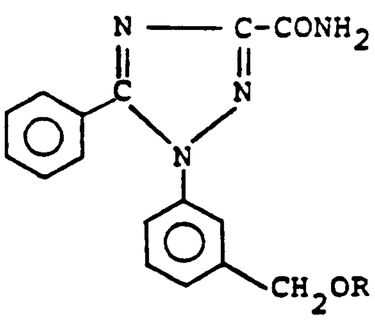

(VI)

dans laquelle R est tel que défini dans la revendication 1, ce procédé comprenant :
a) la réaction d'un dérivé aniline de formule (V)

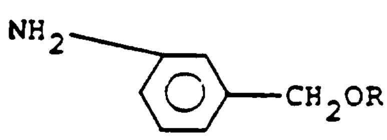

(V)

dans laquelle R est tel que défini ci-dessus, avec de l'acide nitreux, pour donner un sel de

diazonium;
b) la réaction du sel de diazonium obtenu dans l'étape a) avec la 2-phényl-2-oxazoline-5-one, pour donner un composé hydrazone de formule (VII),

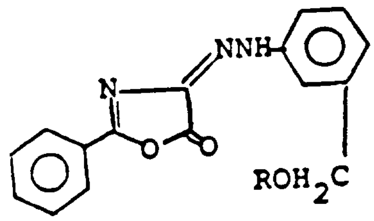

(VII)

dans laquelle R est tel que défini ci-dessus;
c) la réaction du composé hydrazone obtenu dans l'étape b) avec de l'ammoniac; et
d) la déshydratation et la cyclisation du composé obtenu dans l'étape c), pour donner le 1,2,4-triazole-3-carboxamide.

**29.** Procédé selon la revendication 28, dans lequel le dérivé aniline de formule (V) est préparé par réduction d'un composé nitrobenzène de formule (IV)

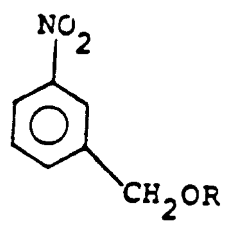

(IV)

dans laquelle R est tel que défini dans la revendication 1.